(19) Europäisches Patentamt
European Patent Office
Office européen des brevets

(11) EP 2 025 349 A2

(12) **EUROPEAN PATENT APPLICATION**

(43) Date of publication:
**18.02.2009 Bulletin 2009/08**

(51) Int Cl.:
***A61K 49/00*** *(2006.01)*

(21) Application number: **08015992.4**

(22) Date of filing: **13.12.2005**

(84) Designated Contracting States:
**AT BE BG CH CY CZ DE DK EE ES FI FR GB GR HU IE IS IT LI LT LU LV MC NL PL PT RO SE SI SK TR**

(30) Priority: **13.12.2004 NO 20045435**

(62) Document number(s) of the earlier application(s) in accordance with Art. 76 EPC:
**05817664.5 / 1 824 522**

(71) Applicant: **GE HEALTHCARE AS**
**Nydalen**
**0401 Oslo (NO)**

(72) Inventor: **Tolleshaug, Helge**
**Postbols 4220 Nydalen**
**Osla N- 0401 (NO)**

(74) Representative: **Canning, Lewis R. et al**
**GE Healthcare Limited**
**Amersham Place**
**Little Chalfont,**
**Bucks. HP7 9NA (GB)**

Remarks:
This application was filed on 11-09-2008 as a divisional application to the application mentioned under INID code 62.

(54) **Fluorescent contrast agents**

(57) The invention relates to contrast agents for imaging of diseases associated with inflammations. More specifically the invention provides optical imaging contrast agents for imaging of activated leukocytes and methods for imaging of such. The contrast agent changes its fluorescent properties upon reaction with oxidants produced by the activated leukocytes.

EP 2 025 349 A2

## Description

### Field of the invention

This invention relates to contrast agents for imaging of diseases associated with inflammations. More specifically the invention provides optical imaging contrast agents for imaging of activated leukocytes and methods for imaging thereof.

### Background of invention

It has been recognised since time immemorial that a fever is usually a sign of an inflammation. An increased sedimentation rate of erythrocytes is another useful sign. During the latter half of the previous century, these indications were further supplemented by *in vitro* tests such as increased levels of C-reactive protein or inflammatory mediators. These tests are very reliable in indicating the presence of an inflammation in the body. However, the location of an inflammation must sometimes be known before it can be treated effectively.

The presence of an occult inflammation may be a very serious clinical problem, for instance in patients who are weak because they are recovering from major surgery.

Inflammatory processes comprise much more than the local redness and abscesses that are universally recognised as inflammation. Inflammation is also involved in many serious and wide-spread diseases that are not usually thought of as inflammatory. Persistent, low-grade infections are now recognised as risk factors for cardiovascular disease. In cancer, inflammation frequently develops around tumours. Persistent inflammatory reactions may lead to fibrosis in some extremely important organs: the liver, the lungs and the heart. Fibrosis reduces the functionality of the tissues in which it occurs and may ultimately lead to organ failure. The immune system is strongly involved in malignant disorders. As regards the atherosclerotic plaque, inflammatory processes play a prominent part in initiation of plaque formation, in the growth of the lesion, and, last by not least, in determining whether the plaque will develop into a stable structure or become an unstable "vulnerable plaque" that may rupture to initiate formation of a potentially lethal thrombus.

Two of the primary and most important features of inflammation are increase in blood flow and loss of endothelial integrity. Within hours, this is followed by an influx of granulocytes, which are the most numerous phagocytosing cells in the blood. A hallmark of inflammation is activation of leukocytes. As a result oxidants are produced.

There are very few options available for localisation of inflammatory foci that are not painful or immediately visible.

WO01/66789 describes compositions and methods for determination of immune response mediators, such as inflammation mediators. The method comprises obtaining a solution containing neutrophils, exposing the neutrophils to a chromophore, allowing the chromophore to oxidize to form a luminescent compound, and measuring the level of visible or ultraviolet radiation. However, the document is directed to an *in vitro* method determining the presence of inflammation mediators in a body fluid, and hence is not applicable to detect a site of inflammation *in vivo.*

Most current methods for detection of sites of inflammation involve taking a blood sample, isolating granulocytes and labelling them. Labelling is usually by means of radioactive isotopes. The labelled compounds may be attached directly to the surface of the cell, they may be attached to monoclonal antibodies to leukocyte antigens, or they may form a complex that is taken up by the cells (e.g. $^{99m}$Tc -hexamethyl-propylene oxime). Finally, labelled compounds, such as labelled chemotactic peptides, may bind to cell-surface granulocyte receptors. The publication by Roddie M. E. et al in Radiology (1988) 166 (3) 767-72, "Inflammation: imaging with 99mTc-HMPAO-labeled leukocytes" describes such methods of detection of inflammations.

Thus, known procedures involve isolation of granulocytes, labelling them and finally re-injecting the cells, allowing them to migrate to the inflammatory focus, and localising the inflammation by means of the radiation. Procedures of this nature are invasive, time-consuming and require some care in order to avoid contamination of the cell suspension. Finally, the object of such procedures is to detect activated cells, as activated cells are those that are immediately involved in the inflammatory process. Avoiding activation of granulocytes or macrophages during the isolation and/or labelling steps requires care and skill; if activated cells are injected, they are likely to be non-specifically retained in the lungs.

Thus, in view of the above considerations there is need for improved contrast agents and methods for imaging of inflammations. The optical imaging contrast agents of the present invention and methods using these contrast agents allow for improved opportunities for detection of sites of inflammation.

### Summary of the invention

An objective of the present invention is to provide contrast agents useful in diagnostic imaging of inflammations. Diseases and indications associated with inflammations include atherosclerotic lesions and cardiovascular diseases. In

addition, inflammatory reactions also occur around some tumours.

A hallmark of inflammation is activation of leukocytes which results in the production of oxidants. It has surprisingly been found that the site of inflammation *in vivo* can be detected by optical imaging procedures. Optical imaging contrast agents that are oxidised by the oxidants created by leukocytes and that change fluorescent properties as a result of the oxidation process, are provided. Changes in the fluorescent properties of the optical imaging contrast agents comprise changes in the excitation or emission wavelengths, changes in the intensity of the fluorescence, or the changes may involve loss of fluorescence.

## Detailed description of the invention

Viewed from one aspect the invention provides an optical imaging contrast agent for imaging of activated leukocytes, wherein the contrast agent upon reaction with oxidants produced by the activated leukocytes changes its fluorescent properties in vivo.

Leukocytes are white blood cells whose function is to destroy foreign substances and invading cells. When referring to leukocytes we mean phagocytes and lymphocytes. Phagocytes include granulocytes, macrophages and monocytes and are capable of phagocytising bacteria and other foreign substances and destroying them. Leukocytes are normally quiescent. For instance, monocytes, lymphocytes and granulocytes circulate in the blood. When leukocytes encounter a pro-inflammatory stimulus, they may become activated. The changes that occur on activation depend on the cell type and the particular stimulus. Certain lymphocytes will, for instance, increase in size and metabolic activity and migrate to lymph nodes. Monocytes and granulocytes will migrate from the blood into surrounding tissue, but while granulocytes have a limited life span, monocytes will differentiate into macrophages to become resident macrophages. Irrespective of the nature of the inflammation , the response is charachterised by the large number of leukocytes at the inflamed site.

During attacks on foreign substances, activated leukocytes produce reactive oxygen species, or oxidants at the site of inflammation. The primary product is superoxide ($O_2^-$) anion, but this molecular species, being reactive, is transformed through enzymatic as well as non-enzymatic reactions, for instance with superoxide dismutase, myeloperoxidase, and iron ions. Exemplary oxidants that are by-products and/or markers of the inflammation cascade include, but are not limited to, oxygen radicals such as hydrogen peroxide ($H_2O_2$), superoxide anion($O_2^-$), oxychloride anion ($OCl^-$), hydroxyl radicals, singlet and triplet oxygen, nitric oxide (NO) and peroxynitrite anion($OONO^-$). One embodiment of the invention is activatable optical imaging contrast agents being oxidised in vivo by any of these oxidants inducing a change in the fluorescent properties of the contrast agent. Many of these oxidants are not radicals in the chemical sense, as they do not possess unpaired electrons, but they do react readily with oxidisable substances, and they are frequently referred to as "oxygen radicals".

In one embodiment of this aspect, the invention provides an optical imaging contrast agent of formula (I),

$$Q\text{-}(L\text{-}R)_p \qquad (I)$$

wherein

- Q represents a fluorochrome in the reduced form,
- R represents a hydrophilic group,
- L represents a linker, and
- p represents and integer of from 1 to 4.

The contrast agent of the present invention comprises a fluorochrome. A fluorochrome is defined as a chemical entity or molecule exhibiting fluorescence, while a fluorophore is defined as a chemical group responsible for fluorescence. A fluorophore is the "core" of a fluorochrome. The fluorochrome, or fluorochrome precursor, comprises a fluorophore or a fluorophore precursor. The fluorochrome fluoresces at certain wavelengths and have extensive conjugated electron systems. Fluorochromes that emit radiation in the visible and near infrared (NIR) spectrum are preferred, and NIR-emitting fluorochromes are most preferred. The fluorochrome comprises a fluorophore that reacts with the oxidants produced by the activated leukocytes and changes it fluorescent properties upon that reaction. In one embodiment, the contrast agents of the invention comprise a reduced form of fluorophores. Preferably the fluorophores are selected from, but are not limited to, the reduced forms of derivatives of naphthalene, anthracene, quinoline, fluoreene, xanthene, acridine, phenanthridine, benzo-oxadizole, pyrrolidone, phenoxazine, styrene, carbocyanine, oxacarbocyanine, indole and isoindole. The contrast agent, accordingly, include fluorophores that are susceptible to oxidation with a concomitant change in fluorescence. As the fluorochrome comprises a reduced fluorophore, the fluorochrome is by definition also in the reduced form.

The contrast agents of the invention comprise fluorochromes that change fluorescent properties at the site of

activated leukocytes as a result of reaction with oxidants. Setsukinai K. et al in The Journal of Biological Chemistry (2003), Vol. 278, No. 5, 3170-3175 describe two fluorescent probes for highly reactive oxygen species. The fluorescence probes undergo oxidative modifications eliminating a part of the molecule, including carbon atoms, when reacting with reactive oxygen species, and hence undergo modifications of the molecular skeleton of the probe.

The type of reaction of the current invention includes oxidation reactions of the contrast agent comprising the reduced fluorochromes. Typically, the reaction involves removal of one or more hydrogen atoms and formation of a double bond that may be part of a system of conjugated double bonds, or the reaction may convert a hydroxyl group to a keto group or aldehyde group. Furthermore, the reaction may involve substitutions of hydrogen atoms with halogen atoms or nitrogen-containing groups such as -NO and $-NO_2$. These substitutions are oxidation reactions in the sense that the substituents draw electrons away from the carbon atoms to which they are attached. All of these are chemical processes that fall within the scope of oxidation reactions in organic chemistry. The type of reaction that is contemplated does not include elimination of part of the carbon skeleton, including heteroatoms such as oxygen, nitrogen and sulphur, of the fluorochrome.

In one embodiment the contrast agents of the invention comprises a fluorochrome selected from the group of fluoresceins, fluorescein derivatives, rhodamines and rhodamin derivatives in the reduced from. Preferably the contrast agent comprises a fluorochrome selected from the group of dihydrofluorescein, dichlorodihydrofluorescein, dihydrorhodamine 123, dihydrorhodamine 6G, dihydrorhodamine 110, dihydrorhodamine 800, dihydrorhodamine B, reduced sulforhodamine B and dihydroethidium (alias dihydroethidine).

Structures of some relevant reduced fluorochromes are shown below.

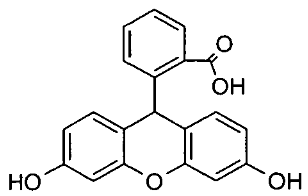

Dihydrofluorescein

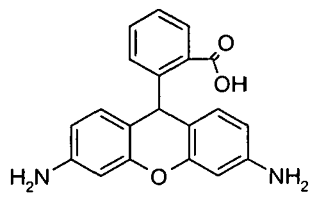

Dihydro-rhodamine 110

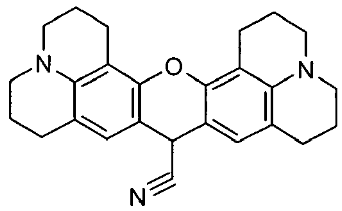

Dihydro-rhodamine 800

An optical imaging contrast agent comprising any of the above fluorochromes is a preferred embodiment of the invention.

The contrast agent further comprises at least one hydrophilic group R attached to the reduced fluorochrome. For the purpose of this invention cell-permeability of the contrast agent is not desirable. Cell permeable molecules are taken up by all cells of the body, unnecessarily increasing the required dose. In inflammatory foci, reactive oxygen species such as superoxide anion are exported from cells of the immune apparatus. Thus, the contrast agent should stay in the extracellular compartment. Cell permeability is usually determined by the ability of a molecule to pass through the hydrophobic core of the lipid double layer of the cell membrane; certain molecules may be carried by physiological transmembrane transport molecules. Cell membrane permeability can be greatly reduced by introducing ionic or hydrophilic groups into the contrast agent. The hydrophilic group R serves to reduce cell permeability and is selected from e.g. the group of sulfonic acid groups, carboxylate groups, carboxyalkyl groups, alkoxycarbonyl groups or alkoxyalky groups, amino groups, sugars, linear or cyclic polyols, and hydrophilic polymers, such as polyethylene glycol or glycans. R may also represent a group comprising both amino acid residues and a PEG-like structure, preferably a bis aminoethyl

ethylene glycol glycine combination. In one embodiment R represents a unit comprised of the monodisperse PEG-like structure, 17-amino-5-oxo-6-aza-3,9,12,15-tetraoxaheptadecanoic acid of formula (II),

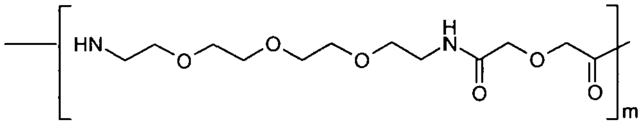

(II)

wherein m equals an integer from 1 to 10 and where the C-terminal is an amide or acid moiety. It is found that the PEG-like structure modifies the pharmacokinetics and blood clearance rates of the contrast agent by effecting a lower uptake of the compounds in tissue i.e. muscle, liver etc. thus giving a better diagnostic image due to less background interference. Such R groups may also be attached in order to modify biodistribution by reducing renal filtration and/or reducing interaction with receptors.

The hydrophilic groups R are attached to the reduced fluorochrome in such away that the fluorescent properties of the fluorochrome are not affected. The R group is preferably placed away from the conjugated fluorophoric system. In fluoresceins, such as in dihydrofluorescein, groups of this nature may be introduced in the 5- or 6-positions without interfering with the fluorescence.

The contrast agents of the invention comprise a linker, L, connecting the fluorochrome with the hydrophilic group. In its simplest form L is a covalent bond or comprises a functional group which permits facile conjugation of the building blocks. L preferably comprises moieties selected from the group including secondary or tertiary amines, amides, thioether, ethers, sulfone, sulfoxide, sulfides, polylactic acid or polyglycolic acid moieties and 1 to 5 amino acids, and alkyl groups. Most preferably L is a covalent bond or an alkyl group, more preferably an $C_{1-6}$ alkyl chain.

Below is an example of how dihydro-rhodamine 800 may be modified with hydrophilic groups. One or more of the groups L-R are positioned such that they do not interfere with the fluorescence of the oxidised fluorochrome.

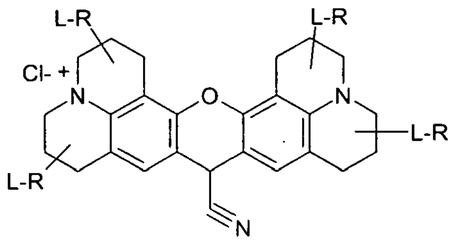

Preferably, the contrast agent is activated to switch on fluorescence when reacting with oxidants, i.e. that the contrast agent, as administered, is non-fluorescent prior to reaction with oxidants and converts to a fluorescent compound on reaction with the oxidant. This includes the alternative that the level of intensity of the fluorescence is changed as a result of the oxidation. Preferably the level of intensity is changed from low to high, e.g. that the contrast agent is almost non-fluorescent prior to oxidation and has a strong fluorescence after oxidation. In this embodiment the change in intensity for the contrast agent is at least 3 times, more preferably at least 5 times and most preferably at least 10 times from before oxidation to after the oxidation. In this regard, as the reduced fluorochrome is preferably non-fluorescent it can be seen as precursor of a fluorochrome. Yet another alternative is that the contrast agents change their excitation/ emission wavelengths upon reacting with oxidants. The primary consideration is that the change in excitation/emission wavelengths must be sufficient to allow distinction between the pre-oxidation and post-oxidation emission using filters or monochromators. Some useful fluorochromes possess emission half-band widths of around 25 nm, and their peaks of excitation/emission may be no more than 10-20 nm apart in some cases; this exemplifies the minimum resolution. In this embodiment the change in excitation/emission wavelengths is at least 10 nm and more preferably more than 20 nm from before oxidation to after the oxidation. Alternatively, changes in the fluorescence emission half-life may be used to distinguish between non-oxidised and oxidised contrast agent if the half-life is changed on oxidation.

Viewed from a second aspect the invention provides contrast agent comprising a reduced fluorochrome, as described in the first aspect, but which are activated in vivo by a two-step mechanism. The two-step mechanism includes

an enzymatic reaction in addition to a reaction with oxidants.

The contrast agents of this aspect are substrates for enzymes, and preferably for hydrolytic enzymes. The contrast agent is thus both a substrate for an enzyme and comprises a fluorochrome or precursor of such. The contrast agents of this aspect are not in themselves capable of reacting with oxidants, but become reactive to such following an enzymatic reaction. Accordingly, the contrast agent undergoes an enzymatic reaction prior to a reaction with oxidants. In this aspect, the fluorochrome of the contrast agent is derivatised with moieties forming a substrate for an enzyme. The derivatising renders the contrast agent susceptible to an enzymatic reaction. Derivatising the fluorochrome also prevents the fluorochrome part of the contrast agent from reacting with oxidants until the enzymatic reaction has taken place. The contrast agents of this aspect are enzyme substrates of formula (III)

$$(R\text{-}L)_r\text{-}Q\text{-}E_n \qquad \text{(III)}$$

wherein Q represents the reduced form of a fluorochrome,

- E represents an entity recognisable by an enzyme,
- n represent an integer from 1 to 3, and
- R represents a hydrophilic group,
- L represents a linker and
- r is an integer of from 0 to 4.

The benefit of contrast agents reacting according to this two-step mechanism is that the oxidation of the fluorochrome is blocked from taking place other than at sites of inflammation. Oxygen radicals are a by-product of the respiratory chain in mitochondria, which contain an abundance of anti-oxidant enzymes. Substances that are susceptible to oxidation may encounter oxygen radicals in the circulation. In order to obtain a contrast agent that is even more specific towards inflammations, it is suggested to use a compound that is not susceptible to oxidation until it has been transformed by action of a specific enzyme. The contrast agent will react with enzymes associated with activated leukocytes before the contrast agent reacts with oxidants and changes its fluorescent properties. Contrast agents of this aspect preferably have an improved specificity towards sites of inflammation.

Relevant enzymes are secreted by activated leukocytes such as macrophages and other phagocytes and are present at sites of inflammation where these are activated. Relevant groups of enzymes useful in this type of application include hydrolases such as peptidases, proteases, phosphatases, sulfatases, glucosidases and esterases. Other useful enzymes include dehydrogenases, oxidases, reductases, kinases, transferases and ligases. The contrast agent comprises a reduced fluorochrome (Q), or precursor of such, derivatised with a moiety E preferably forming an enzyme substrate for any one of these enzymes.

E is preferably selected from the group of phosphates, sugars and peptides to provide contrast agents being enzyme substrates for phosphatases, glucosidases and peptidases.

A phosphatase substrate is obtained when E is a phosphate moiety and this is attached to the fluorochrome, preferably to the hydroxyl groups of a reduced fluorochrome, such as dihydrofluorescein. An example of a substrate for phosphatase is shown below wherein two phosphate groups are linked to the reduced fluorochrome:

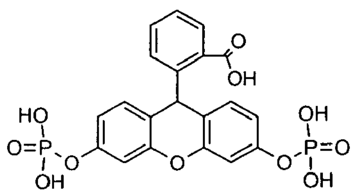

Dihydrofluoresceine diphosphate

Substrates for glucosidases are obtained by attaching specific sugars (E) by glycosidic linkage to fluorochromes; for instance, a substrate for N-acetylglucosaminidase may be synthesised by attaching N-acetylglucosamine by a glycosidic linkage to the hydroxyl groups of dihydrofluorescein. Other relevant sugars are galactose, N-acetylgalactosamine, mannose and fucose. A substrate for galactosidase is shown below:

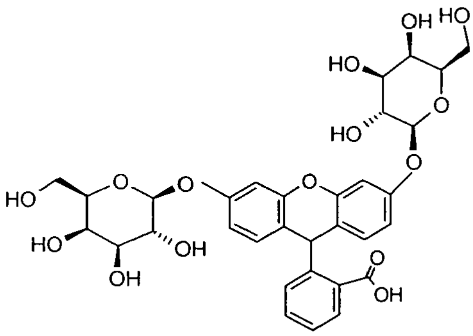

Bis(β-galactosyl)-dihydrofluorescein

A contrast agent according to this aspect being a peptidase substrate is accordingly obtained by attaching a peptide (E) moiety to a reduced fluorochrome, such as to the amino groups of reduced rhodamine 110. The peptide is selected from any peptides which may react with a peptidase by cleavage of the peptide from the reduced fluorochrome comprising from 1 to 5 amino acids. The peptide may contain a selection of the 20 natural amino acids or it may contain modified or unusual amino acids. The amino acids may be joined by isopeptide bonds and/or the natural peptide bonds between α-amino groups and α-carboxyl groups. The peptide can be synthesized using known methods of chemical synthesis and particularly useful is the solid-phase methodology of Merrifield employing an automated peptide synthesizer (J. Am. Chem. Soc., 85: 2149 (1964)). In addition, coupling of the fluorochrome to the peptide can also be carried out automatically employing an automated peptide synthesizer yielding an amide bond between the peptide and the fluorochrome. Synthesis of peptides by solid phase techniques is based upon the sequential - addition of protected amino acids linked, optionally through a linker group, to a solid phase support. In one commonly employed method, the α-amino group is suitably protected with acid labile or base labile protecting groups. Following addition and coupling of the first amino acid residue, the α-amino protecting group is removed.

The reduced fluorochrome Q is selected from the same type of fluorochromes as mentioned for the first aspect, and is preferably selected from the group of dihydrofluorescein, dichlorodihydrofluorescein, dihydrorhodamine 123, dihydrorhodamine 6G, dihydrorhodamine 110, dihydrorhodamine 800, and dihydroethidium. With regard to this aspect a preferred fluorochrome is dihydrorhodamine 123, as it has two amino groups that may be derivatised with an entity recognisable by an enzyme to form contrast agents according to this aspect.

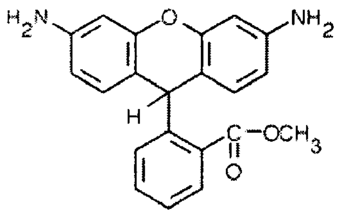

Dihydrorhodamine 123

Other dyes than those mentioned above may be used. Derivatisable fluorochromes that are useful *in vivo,* e.g. dyes with excitation and emission wavelengths in the near infrared are preferred. The fluorochromes are susceptible to oxidation in vivo and change the fluorescence as a result of the oxidation.

n is an integer from 1 to 3. For fluorochromes comprising more than one group which may be modified with an entity recognisable by an enzyme, it is preferred that all these groups are modified. Examples of preferred groups of the fluorochrome that can be derivatised are hydroxyl groups and amino groups.

The contrast agents of this aspect optionally comprise a hydrophilic group R as in the first aspect, linked to the fluorochrome by a linker. Cell membrane permeability can be greatly reduced by introducing ionic or hydrophilic groups into the contrast agent. The hydrophilic group R and the linker are selected from the same groups as for the first aspect.

A general example of a contrast agent and the two-step mechanism of this aspect is shown below.

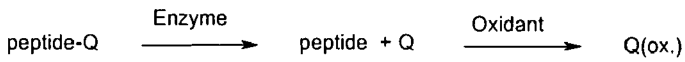


In this sketch, peptide-Q denotes a contrast agent according to this aspect of the invention, wherein the contrast agent is a substrate for an enzyme, e.g. for a peptidase. Q denotes the reduced, i.e. non-oxidised form of the chromophore, while ox. denotes the oxidised form. The contrast agent comprises a peptide consisting of one or more amino acids and may also include blocking groups, as used in peptide chemistry. The peptide prevents the fluorochrome from being oxidised. Upon reaction with the enzyme, the peptide will be cleaved off from the fluorochrome, liberating the reduced fluorochrome. The reduced fluorochrome subsequently reacts with oxidants and changes its properties with regard to fluorescence when the oxidised form is generated.

Cathepsin B is an enzyme that cleaves peptides at the carboxyl end of arginine or lysine. A typical contrast agent substrate according to this aspect for reaction with cathepsin B comprises the group $(B'\text{-Arg})_n\text{-Q}$, wherein the entity recognisable by an enzyme (E) consist of arginine linked to B', wherein B' represent an aminoterminal blocking group, Q represent a reduced fluorochrome and n represent an integer from 1 to 3. For instance, one or both of the amino groups of dihydrorhodamine 110 may be derivatised with a B'-Arginine moiety, thus creating a substrate for cathepsin B. Upon reaction with the enzyme, the arginine will be removed, leaving a free reduced fluorophore. An example of such substrate for cathepsin B is shown below wherein B' is a benzoyl group.

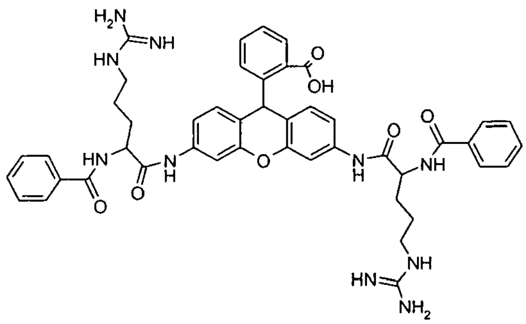

Bis-(*N*-benzoyl-arginine)-dihydrorhodamine 110

In a second step, the reduced fluorochrome Q will react with oxidants formed as a function of an inflammation process. The two-step mechanism taking place for this contrast agent is shown below.

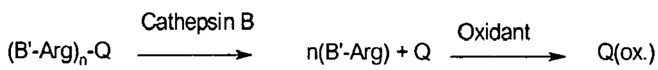


In a further aspect, the invention provides processes for the preparation of contrast agents according to the invention. The contrast agents according to the invention comprise a reduced fluorochrome. The reduced form of a fluorochrome is produced by reaction of a fluorochrome with a reducing agent. The reduction reaction typically involves the addition of one or more hydrogen atoms to the fluorochrome, so that double bonds are converted to single bonds. The double bond is usually situated between two carbon atoms or between a carbon atom and an oxygen or nitrogen atom. Chemical reduction of fluorescein and rhodamin-based fluorochromes yields colorless and nonfluorescent dihydrofluoresceins and dihydrorhodamines. Such reduced fluorochromes may be prepared from commercially available fluorochromes such as fluorescein, rhodamine 110, rhodamine 123, rhodamine 6G, rhodamine 800, rhodamine B, reduced sulforhodamin B, ethidium or cresyl violet and reacting these with a reducing agent such as e.g. sodium dithionite, sodium borohydride, sodium cyanoborohydride, lithium dimethylaminoborohydride or sodium triacetoxyborohydride. Rhodamine 110 may be reduced by zinc powder in dilute acetic acid. Very powerful reducing agents such as lithium aluminium hydride or diborane should be used with caution as they may lead to elimination of substituents. The reduced fluorochromes are preferably non-fluorescent.

The hydrophilic groups R may be attached to the fluorochrome either before reduction of the fluorochrome or preferably to an already reduced fluorochrome. Fluorochromes comprising reactive groups, such as amine groups and hydroxyl groups, that should not be modified, should be protected. An amine functionality may be protected by esters, suitably $C_{1-6}$ alkyl or $C_{1-6}$ haloalkyl esters, preferably acyl esters such as t-butoxycarbonyl (Boc), or ethers, preferably $C_{1-6}$ alkyl ethers, or as amides suitably alkylamides such as formyl amide. A hydroxyl group may be protected as an ether, such as a silyl ether or alkyl ether, or as an ester. These protecting groups may be conveniently removed by hydrolysis, for example in presence of a acid or base. Reaction with a hydrophilic group, such as with an amine, may be obtained using an activated hydroxyl group, isothiocyanat or sulfonylchloride. For attachment to sulfohydryl groups maleimides or chloroketones may e.g. be used.

For preparation of contrast agents comprising an entity recognisable by an enzyme, the fluorochromes may be modified to include substituents such as peptides, carbohydrates or phosphate groups. The peptides may be introduced by reaction of activated esters of peptides that may comprise protecting groups on amines or sulfhydryl groups in order to eliminate side reactions. Carbohydrates may be introduced as activated derivatives, for instance chloroacetimidates, that include protecting groups on the hydroxyls, usually acetyl groups, as disclosed by Marjan Jeselnik et al.. 2000. "Novobiocin-related compounds", Carbohydrate Research 328 (2000). 591-597.Phosphate groups may be introduced by reacting a suitably protected fluorochrome with diphenyl chlorophosphonate, and then removing the phenyl groups by hydrogenation over palladium and hydrolysis, as disclosed by Lardy Henry A. and Hermann O.L. Fischer. 1946. "Phosphoric esters of biological importance. I. The synthesis of glucose-6-phosphate". J.Biol.Chem. 164 (2) 513-9.

A further aspect of the invention is a method of detecting activated leukocytes in a tissue by in vivo optical imaging, the method comprising;

i) administering an optical imaging contrast agent to the subject, wherein the contrast agent, by reaction with oxidants produced by the activated leukocytes, changes its fluorescent properties,
ii) detecting the change of fluorescent properties from the contrast agent.

The contrast agent administered in step i) of the method has the properties as described for the first aspect or second aspect of the invention. Accordingly, the contrast agent comprises a reduced fluorochrome, or a precursor of such, changing its fluorescent properties when reacting with oxidants. Further, step i) optionally includes a two-step mechanism wherein the contrast agent acts as a substrate for an enzyme, undergoing an enzymatic reaction prior to the reaction with the oxidants.

A preferred method includes generating an image of a site of a human or animal body by optical imaging detecting fluorescence, involving administering a contrast agent as described to said body, e.g. into the vascular system, and generating an image of at least a part of said body, to which the contrast agent has distributed. The method further preferably includes detection of sites of inflammation based on detection of fluorescence. The change of fluorescent property is either a change in the appearance or disappearance of fluorescence, a change in the emission and/or excitation wavelength, a change in the level of intensity of fluorescence, or a change in the fluorescence lifetime. Such detection of fluorescence may include measurements of the level of fluorescent, or the identification of sites of inflammation is based on detection of a distinction in the excitation/emission wavelengths between non-oxidised and oxidised contrast agent or the detection includes measurements of the fluorescence lifetime.

Viewed from a still further aspect the invention provides a method of generating enhanced images of a human or animal body by imaging, previously administered with a contrast agent as defined, which method comprises generating an image of at least part of said body.

The present invention also provides a pharmaceutical composition comprising an effective amount, e.g. an amount effective for enhancing image contrast in in vivo imaging of a contrast agent of the invention, or a salt thereof, together with one or more pharmaceutically acceptable adjuvants, excipients or diluents.

The contrast agents, or compositions thereof, of the invention are intended for use in *in vivo* optical imaging. More preferably, the contrast agents are used for imaging and detection of diseases and indications associated with inflammations. Such indications include atherosclerotic lesions, particularly those that contain activated macrophages and consequently are unstable ("vulnerable" or "culprit" lesions). Further indications include inflammatory reactions such as those occurring around some tumours, low-grade infections being a risk factor for cardiovascular diseases, and persistent inflammatory reactions leading to fibrosis.

Viewed from a further aspect the invention provides the use of a contrast agent of the invention for the manufacture of a contrast enhancing agent for use in a method of diagnosis of inflammations involving administration of said contrast enhancing agent to a human or animal body and generation of an image of at least part of said body.

The present invention will now be further illustrated by way of the following nonlimiting examples.

## Examples

<u>Example 1. Synthesis of dihydro-rhodamine 800 and re-oxidation.</u>

To 2 ml of a 1 mM solution of rhodamine 800 (Fluka, prod. No. 83701) in 0.1 M borate buffer, pH 8.5 was added approximately 5 mg of sodium borohydride. The mixture was shaken and left at room temperature for two hours. 40 microliters of the reduced solution was added to 960 μl of phosphate-buffered saline, pH 7.4. Figure 1A shows the spectrum of the reduced rhodamine flourochrome, i.e. dihydro-rhodamine 800, (*dotted line*) which was recorded on a Hewlett-Packard diode array spectrophotometer. The spectrum of untreated rhodamine 800 (*solid line*) was recorded by exactly the same procedure. The sample of diluted, reduced fluorochrome was gently oxidized in air and its spectrum recorded (*dashed line*).

Figure 1 B shows the formulas for rhodamine 800 and dihydro rhodamine 800.

The example shows that rhodamine 800 may be reduced to dihydro-rhodamine 800, losing nearly all absorption in the visible region. In order to excite fluorescence, light must be absorbed by the fluorochrome. As there is no absorption in the visible region, there is no fluorescence from reduced rhodamine 800 following exposure to visible light. The reduced fluorochrome was oxidised, regaining its optical properties.

Figure 1A.

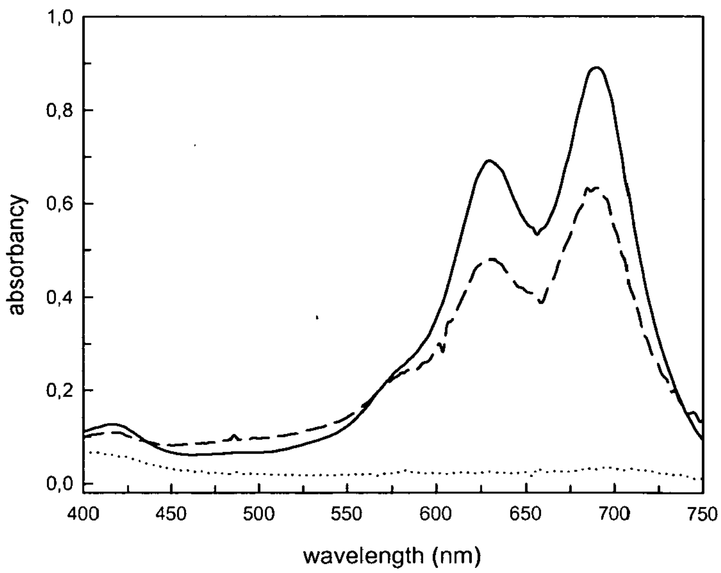


Figure 1B:

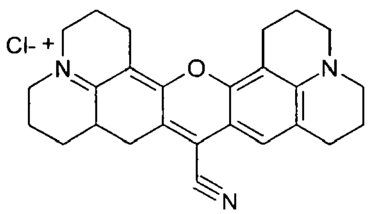

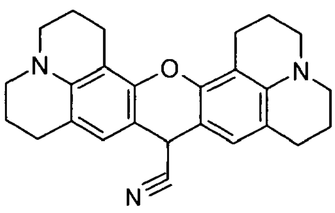

Rhodamine 800

Dihydro-rhodamine 800

<u>Example 2. Synthesis of dihydro-rhodamine 110 and re-oxidation.</u>

To 2 ml of a 1 mM solution of rhodamine 110 (Fluka, prod. No. 83695) in 0.1 M borate buffer, pH 8.5 was added approximately 5 mg of sodium borohydride. The mixture was shaken and left at room temperature for two hours. 40

microliters of the reduced solution was added to 960 µl of phosphate-buffered saline, pH 7.4. Figure 2A shows the spectrum of the reduced rhodamine flourochrome, i.e. dihydro-rhodamine 11, (*dotted line*) which was recorded on a Hewlett-Packard diode array spectrophotometer. The spectrum of untreated rhodamine 110 (*solid line*) was recorded by exactly the same procedure. The sample of diluted, reduced fluorochrome was gently oxidized in air and its spectrum recorded (*dashed line*).

Figure 2B shows the formulas for rhodamine 110 and dihydro-rhodamine 110.

The example shows that rhodamine 110 may be reduced to dihydro-rhodamine 110. In order to excite fluorescence, light must be absorbed by the fluorochrome. As there is virtually no absorption in the visible region, there is very little fluorescence from reduced rhodamine 110 following exposure to visible light. However, the reduced fluorochrome was oxidised, regaining its optical properties.

Figure 2A.

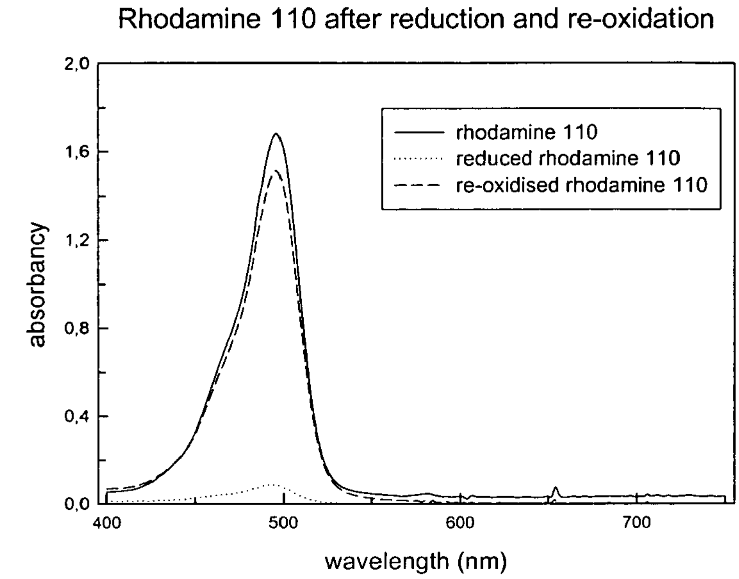


Figure 2B:

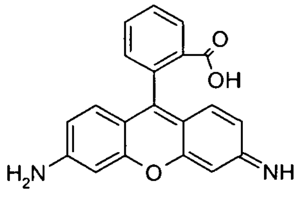

Rhodamine 110

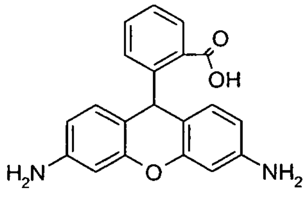

Dihydro-rhodamine 110

Example 3. Synthesis of dihydrofluorescein and re-oxidation

To 2 ml of a 1 mM solution of fluorescein (Sigma, prod. No. F-6377) in 0.1 M borate buffer, pH 8.5 was added approximately 5 mg of sodium borohydride. The mixture was shaken and left at room temperature for two hours. 40 microliters of the reduced solution was added to 960 µl of phosphate-buffered saline, pH 7.4. Figure 3A shows the spectrum of the reduced fluoroescein flourochrome, i.e. dihydrofluorescein (*dotted line*) which was recorded on a Hewlett-Packard diode array spectrophotometer. The spectrum of untreated fluorescein (*solid line*) was recorded by exactly the same procedure. The sample of diluted, reduced fluorochrome was gently oxidized in air and its spectrum recorded

(*dashed line*). Figure 3B shows the formulas for fluorescein (fluorescent) and dihydrofluorescein (non-fluorescent).

The example shows that fluorescein may be reduced to dihydrofluorescein, losing nearly all absorption in the visible region. In order to excite fluorescence, light must be absorbed by the fluorochrome. As there is no absorption in the visible region, there is no fluorescence from reduced fluorescein following exposure to visible light. However, the reduced fluorochrome was oxidised, regaining its optical properties.

Figure 3A.

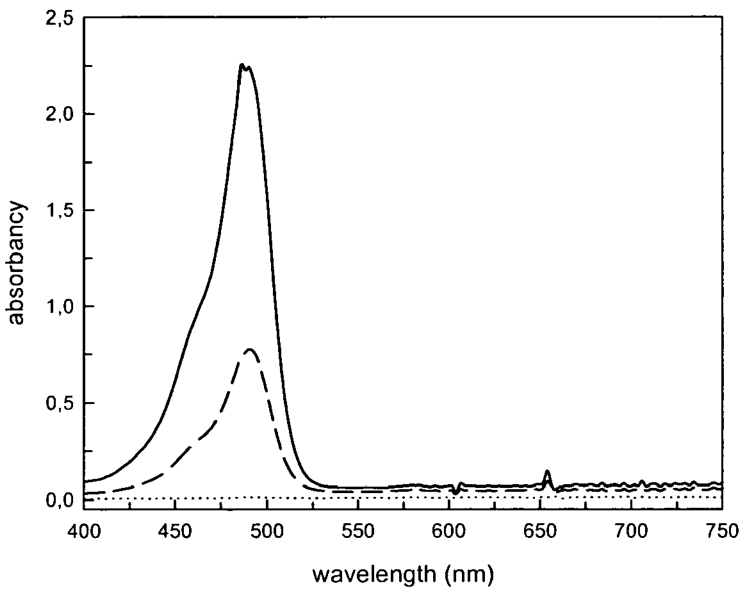


Figure 3 B.

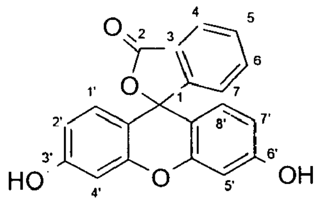

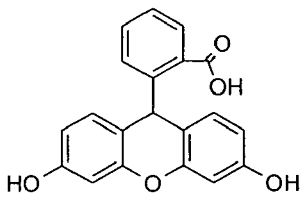

Fluorescein

Dihydrofluorescein

Example 4. Oxidation of reduced rhodamine 110 by hydrogen peroxide.

Rhodamine 110 was reduced as in Example 2. Hydrogen peroxide to a final concentration of 10 μM was added to a portion of the solution of reduced rhodamin 110, i.e. to dihydrorhodamin 110, and the increase in fluorescence was recorded at 538 nm (excitation at 485 nm) on a Thermo Fluoroscan Ascent FL fluorimeter. The experiment shows, as given in Figure 4A, that reduced rhodamine 110 is rapidly oxidised by a low concentration of hydrogen peroxide. Figure 4B shows the formulas of dihydrorhodamin 110 and rhodamin 110

Figure 4A.

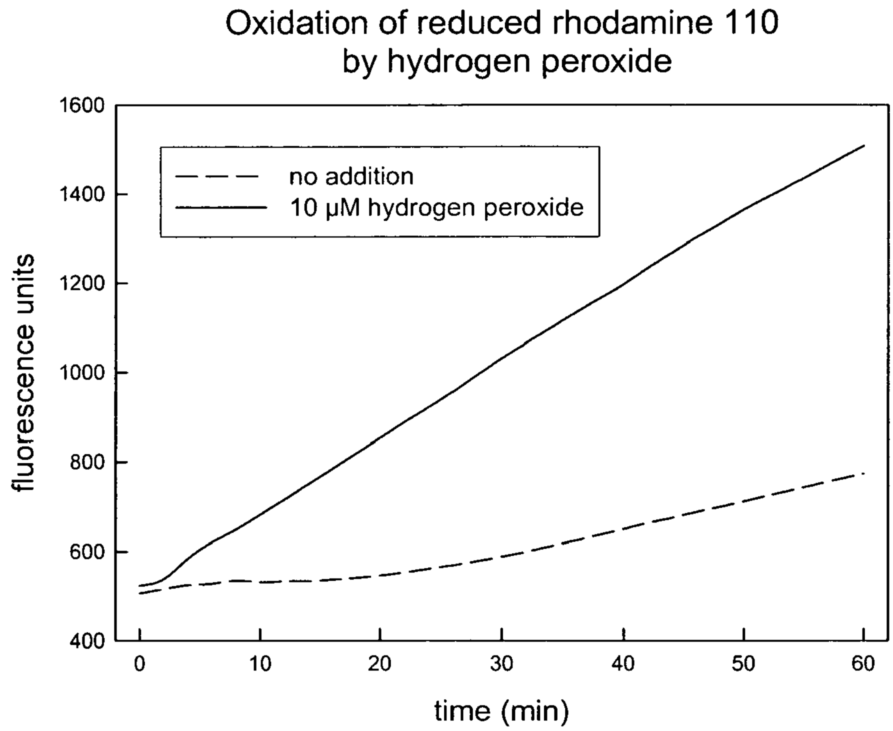


Figure 4B.

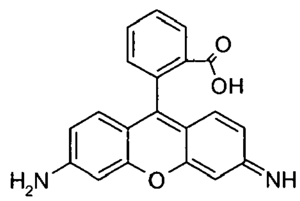

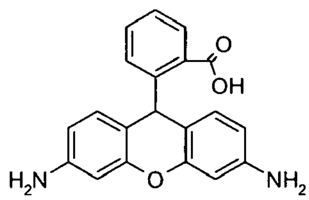

Rhodamin 110 Dihydrorhodamine 110

**Claims**

1. An optical imaging contrast agent of formula of formula (IIIa):

$$E_n\text{-}Q\text{-}(L\text{-}R)_p \qquad \text{(IIIa)}$$

wherein:

E represents an enzyme substrate;
n represents an integer of value 1 to 3;
Q represents a fluorochrome in the reduced form which upon reaction of said contrast agent with oxidants produced by activated leukocytes changes its fluorescent properties *in vivo,*
R represents a hydrophilic group,
L represents a linker,
p represents an integer of value 1 to 4.

2. The optical imaging contrast agents of claim 1 wherein Q is selected from a fluorescein or rhodamine in the reduced form.

3. The optical imaging contrast agent as claimed in claims 1 or 2 wherein Q is selected from the group of dihydrofluorescein, dichlorodihydrofluorescein, dihydrorhodamine 123, dihydrorhodamine 6G, dihydrorhodamine 110, dihydrorhodamine 800, dihydrorhodamine B, reduced sulforhodamine B and dihydroethidium.

4. The optical imaging contrast agent as claimed in any one of claims 1 to 3 wherein R is selected from the group of sulfonic acid groups, carboxylate groups, carboxyalkyl groups, alkoxycarbonyl groups or alkoxyalkyl groups, amino groups, sugars, linear or cyclic polyols, and hydrophilic polymers.

5. The optical imaging contrast agent of any one of claims 1 to 5 wherein the change of fluorescent property is a change in the appearance or disappearance of fluorescence, a change in the emission and/or excitation wavelength, a change in the level of intensity of fluorescence, or a change in the fluorescence lifetime.

6. The optical imaging contrast agent of claims 4 or 5 wherein a reaction of the contrast agent with oxidants involves a formation of one or more double bonds, conversion of a hydroxyl group to a keto- group or a aldehyde- group or substitution of hydrogen atoms with halogens atoms or nitrogen-containing groups.

7. The optical imaging contrast agent of any one of claims 1 to 6 which is activated *in vivo* in a two-step mechanism including an enzymatic reaction in addition to changing its fluorescent properties upon reaction with oxidants.

8. The optical imaging contrast agent of any one of claims 1 to 7, where E is a substrate for enzymes selected from the group of: peptidases, proteases, phosphatases, sulfatases, glucosidases, esterases, dehydrogenases, oxidases, reductases, kinases, transferases and ligases.

9. A method of detecting activated leukocytes in a tissue by *in vivo* optical imaging, the method comprising:

i) administering the optical imaging contrast agent of any one of claims 1 to 8 to the subject;
ii) detecting the change in fluorescent properties from the contrast agent.

10. The method of claim 9, wherein E is a substrate for hydrolytic enzymes, and wherein the contrast agent undergoes a enzymatic reaction prior to the reaction with the oxidants.

11. A pharmaceutical composition comprising the contrast agent as claimed in any one of the claims 1 to 8, together with one or more pharmaceutically acceptable adjuvants, excipients or diluents.

12. Use of the contrast agent as claimed in any one of claims 1 to 8 in the manufacture of a contrast enhancing agent for use in the diagnosis of inflammation.

13. Contrast agents of any one of the claims 1 to 8 for use in diagnostic imaging for detection of sites of inflammation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0166789 A


### Non-patent literature cited in the description


- **RODDIE M. E. et al.** Inflammation: imaging with Tc-HMPAO-labeled leukocytes. *Radiology,* 1988, vol. 166 (3), 767-72
- **SETSUKINAI K. et al.** *Journal of Biological Chemistry,* 2003, vol. 278 (5), 3170-3175
- *J. Am. Chem. Soc.,* 1964, vol. 85, 2149
- **MARJAN ; JESELNIK et al.** Novobiocin-related compounds. *Carbohydrate Research,* 2000, vol. 328, 591-597
- **LARDY HENRY A. ; HERMANN O.L. FISCHER.** Phosphoric esters of biological importance. I. The synthesis of glucose-6-phosphate. *J.Biol.Chem.,* 1946, vol. 164 (2), 513-9